# EUROPEAN PATENT SPECIFICATION

(11) **EP 3 991 651 B1**
(45) Date of publication and mention of the grant of the patent: **19.07.2023**
(21) Application number: 20204788.2
(22) Date of filing: 29.10.2020
(51) Int. Cl.: A61B 5/15, A61B 5/151, A61B 5/157

(54) **HAND OPERATED ACTUATOR MECHANISM**
HANDBETÄTIGTER AKTUATORMECHANISMUS
MÉCANISME D'ACTIONNEMENT MANUEL

(43) Date of publication of application: 04.05.2022
(73) Proprietor: Homedicus GmbH, 12103 Berlin (DE)
(72) Inventor: Kücüktas, Elif, Berlin (DE); Diebold, Michael, Berlin (DE); Riester, Markus, Wiesbaden (DE)
(74) Representative: Eisenführ Speiser

(56) References cited:
- WO-A1-98/48696
- WO-A1-2005/009238
- WO-A1-2005/110227
- WO-A1-2006/110573
- WO-A1-2009/025395
- US-B1- 6 248 120

## Description

The present invention is directed to a hand-operated actuator mechanism, to a piercing device comprising said actuator mechanism, to a testing device comprising said piercing device and to methods for operating the actuator mechanism and the testing device.

US 2012/0277697A1 describes systems and methods for delivering to and/or receiving fluids or other materials, such as blood or interstitial fluid, from subjects, e.g. from the skin. In a particular embodiment, when an actuator is actuated by a user, a piercing element is engaged and driven toward the skin.

WO 2005/009238 A1 and US 6,248,120 B1 disclose a puncturing device designed particularly for puncturing a patient's skin in order to collect a blood sample for diagnostic purposes. The puncturing has and a puncturing needle, a driving spring a return spring and a push button with guiding arms.

WO 2009/025395 A1 discloses a puncture instrument comprising a needle for instantaneously projecting the needle to puncture the human skin therewith, wherein a needle assembly is pushed directly by the push rod and thus not mediated with an elastic body for axially biasing the needle assembly.

WO 2005/110227 A1 discloses a lancet device with a lancet structure that is disposed within a housing and adapted for movement between a retaining or pre-actuated position wherein the puncturing element is retained within the housing, and a puncturing position wherein the puncturing element extends through a forward end of the housing. The lancet device includes a drive spring disposed within the housing for biasing the lancet structure toward the puncturing position, and a retaining hub retaining the lancet structure in the retracted position against the bias of the drive spring.

It is an object to provide an improved or alternative piercing device.

According to the invention, a hand-operated actuator mechanism is provided. The actuator mechanism is configured for driving an external piercing element with a predetermined force. The actuator mechanism comprises a mounting element, a trigger element and a piercing element carrier. The piercing element carrier can be operatively connected to the piercing element.

According to a first aspect of the actuator mechanism, the trigger element is connected to the mounting element via at least one first breakable connection element, i.e. a connection element that is designed to break in a controlled manner when a predetermined force is applied. The trigger element further comprises or is connected to a pushing member that can be pushed with a human finger. Via the pushing element a force can be exerted on the trigger element. The first breakable connection element is configured to break if a predetermined pushing finger force is pushingly applied to said pushing member. Further, the piercing element carrier is connected to the trigger element via at least one second breakable connection element. The second breakable connection is designed to break in a controlled manner when a second predetermined force is applied. The piercing element carrier is movable between an initial position wherein the first and the second breakable connection elements are unbroken, and a stop position wherein an abutment stops a movement of the piercing element carrier.

Applying a force on the pushing member in a longitudinal direction causes a stress in the material of the first breakable connection element, e.g. tensile and/or shear stress. The first breakable connection element is advantageously configured to break upon application of the predetermined pushing finger force. Once the first breakable connection element is broken, the trigger element and the piercing element carrier are free to move independently of the mounting element in at least the longitudinal direction. The free-moving part of the actuator mechanism, which includes at least the trigger element and the piercing element carrier is accelerated with an acceleration that depends primarily on the predetermined pushing force needed to break the first breakable connection element and on the mass of the accelerated objects, for instance the trigger element, the pushing element, the piercing element carrier, and external piercing element and the finger.

Due to the first breakable connection element, a predefined piercing force can be achieved and safety is increased since the actuator can only be used once in its intended form thus ensuring a single-use operation.

In the following, embodiments of the actuator mechanism of the first aspect of the invention are described.

In a preferred embodiment of the actuator mechanism, that further increases the safety, the second breakable connection element is configured to be broken when the piercing element carrier reaches the stop position. This enables a separation of the trigger element from the piercing element carrier so that a further actuation of the pushing member is uncoupled from the piercing element carrier, thus ensuring that no further force can be transferred from the actuator to the piercing element carrier.

In order to further increase the safety, an embodiment of the actuator mechanism further comprises an elastic element that is arranged to act on the piercing element carrier for returning the piercing element carrier form the stop position in the direction of the initial position. Thus, the elastic element is configured to exert a retraction force for driving the piercing carrier element towards the initial position. In an embodiment wherein the second breakable connection is configured to be broken when the piercing element carrier reaches the stop position, the piercing element carrier is advantageously driven towards the initial position independently on whether further pushing finger force is applied on the pushing member, thus guaranteeing a retraction of the piercing element carrier. Suitable elastic elements include, but are not limited to, coil springs, which can be conical, or more preferably cylindrical, or strips such as metal or plastic strips, elastics bands such as rubber bands, etc.

In an embodiment, the abutment (that is arranged to stop the movement of the piercing element carrier once the first breakable connection element is broken) is a non-movable element, with respect to the mounting element. In a preferred alternative embodiment, the abutment is the elastic element in its stressed or compressed state, for instance a helical spring in its fully compacted, solid state. The application of the predetermined pushing finger force on the pushing element in the longitudinal direction causes the breakage of the first breakable connection element and an acceleration of the trigger element and the piercing carrier element, which are linked by the second breakable connection element, towards the stop position. This accelerated movement is carried out against the elastic element, which is moved away from its initial position, storing elastic potential energy and decelerating the trigger element and the piercing carrier element until it stops. Thus, the elastic strip or band in a stressed state or the coil spring in the compressed state acts as the abutment that stops the movement of the piercing element carrier.

In an embodiment, the elastic element, such as a coil spring, is further arranged and configured to break the second breakable connection elements when the stop position is reached. For instance, the elastic element is arranged so that in applies a breaking force on the second breakable connection element when the stop position is reached. In an alternative embodiment, the actuator mechanism further comprises a non-movable breaking element that is configured to break the second breaking connection element. In an embodiment, the coil spring, preferably a cylindrical coil spring is arranged as a guide for an external piercing element attachable to the piercing element carrier to ensure that the piercing element moves in a longitudinal direction of the coil spring and preferably inside the coils. In an embodiment where the diameter of the coil spring is smaller than the dimensions of the piercing carrier element, the actuator mechanism includes an additional breaking element arranged to be in contact and to break the second breakable connection element.

In a particular embodiment of the actuator mechanism, which may include any of the technical features described above, the first breakable connection is adapted to be broken upon exertion of a pushing finger force of 20-100 N on the pushing member. Preferably, the pushing finger force necessary to break the first breakable connection element is between 30 and 80 N and more preferably between 35 and 55 N. The actual value of the predetermined pushing finger force that is to be applied for breaking the first breakable connection element can be selected in dependence on the application or the intended user. For example, predetermined pushing finger forces between 50 and 100 N provides an increase safety feature that prevents an accidental activation by children. Actuator mechanism intended for use by elderly people may advantageously require less pushing finger force than those intended to you by younger adults.

According to a second aspect of the present invention, a piercing device is described. The piercing device comprises a peripheral support structure forming an inner volume. It also comprises a hand-operated actuator mechanism in accordance with any of embodiments of the first aspect of the invention, wherein the mounting element of the actuator mechanism is connected to the peripheral support structure.

Preferably, the abutment and/or the elastic element is connected to the peripheral support structure.

An embodiment of the piercing element comprises a piercing element connected to the piercing element carrier. In an alternative embodiment, the piercing element carrier comprising connecting means for connecting an external piercing element. Suitable piercing element include, but are not limited to, lancets, needles, hollow needles, trocars and catheters.

In a preferred embodiment of the piercing device, the piercing element is arranged to remain within the inner volume at the initial position and to protrude from the inner volume at the stop position. This reduces the risk of accidental damage by the piercing element before the pushing member is actuated.

Preferably, the piercing device is a hand-held device. In a particular embodiment, the peripheral support structure has a cylindrical shape defined by a height value and a diameter value, and wherein the diameter value is larger than the height value. Preferably, the height of the peripheral support structure is smaller than 5 cm, more preferably smaller than 3 cm and even more preferably smaller than 2.5 cm. The diameter of the peripheral support structure is preferably smaller than 5 cm.

According to a third aspect of the present invention, a testing device is described. The testing device comprises a piercing device according to any of the embodiments of the piercing device of the second aspect of the present invention. The testing device further comprises a fluid sample receiving unit for receiving a fluid sample for testing and at least one fluid transport element having an input end fluidly connected to the fluid sample receiving unit. The fluid transport element is arranged and configured to transport a fluid away from the input end. The testing device also comprises at least one testing unit in fluidic communication with the fluid transport element. The testing unit comprises a respective reacting material configured to react in a predetermined manner to a pre-specified analyte or property of the fluid.

In an embodiment, the fluid sample receiving unit is or comprises a piercing element. In another embodiment, the fluid sample receiving unit comprises connection means for attaching an external piercing element. In another embodiment, the sample fluid receiving unit is an interface for receiving a fluid sample.

In a preferred embodiment, the testing device is a device suitable for performing a lateral flow assay. Also known as lateral flow immunochromatographic assays. These assays are typically intended to detect the presence of a target substance in a fluid sample and are widely used in medical diagnostics for home testing, point of care testing, or laboratory use.

In a particular embodiment, the fluid transport element is a capillary wick or other capillary bed having the capacity of transporting a fluid spontaneously, for instance by capillary effect. Alternatively, the fluid transport element is a micro-fluidic system. In another embodiment, the fluid transport element includes both a capillary bed or wick and a micro-fluidic system.

In an embodiment, each of the testing units comprises a different reacting material configured to react in a pre-specified manner to a respective pre-specified analyte. Alternatively, two or more of the testing units comprise one or more test portions having a given reacting material with a same or a respective different sensitivity, either in order to improve the accuracy of testing assembly or in order to enable a semi-quantitative evaluation of the given analyte.

In a preferred embodiment, that can be arranged as a hand-held testing device with reduced dimensions, the fluid transport element has, in a planar state, a center line length, a width and a thickness that is shorter than the center line length and the width. Also, a width direction of the fluid transport element extends at an angle smaller than 90° with respect to a normal of a support plane defined by the peripheral support structure. In this particular embodiment, the fluid transport element is curved, resulting in a shortest distance between two opposite longitudinal ends of the fluid transport element being shorter than the center line length in the planar state.

Preferably, the fluid transport element is a capillary bed or wick that has two flat sides that are spaced apart by the thickness of the given fluid transport element. In a planar state, the fluid transport element has a length, a width and a thickness.

If the fluid transport element is rectangular (neglecting its thickness) in its planar state, then the fluid transport element has a given center line length in a longitudinal direction, a given width in a width direction perpendicular to the longitudinal direction and a given thickness in a thickness direction perpendicular to both the longitudinal direction and the width direction, that is shorter, i.e., has a smaller extension, than the center line length and the width. In this particular case of a rectangular fluid transport element, a length of the fluid transport element and a length of a center line in the middle of the fluid transport element, hereinafter also referred to as center line length, is equal to a length of the longitudinal edges of the fluid transport element's flat surfaces.

Alternatively, the edges of the flat sides may be curved, (i.e. not straight) in the planar state. This results in a fluid transport element having a curved shape in its planar state. In this case, the center line length is the length of a center line located midway between the longitudinal edges of the fluid transport element. The center line length is inherent to the testing strip and independent on an actual state - curved or planar - of the fluid transport element.

The distance between the longitudinal ends of a planar, curved fluid transport element may be shorter than the length of the center line.

To further limit the outer dimensions of the fluid transport element - and thus the envelop of the fluid transport element - the fluid transport element may be arranged non-planar, i.e. bent or further curved in a third dimension. This includes for example fluid transport elements having straight longitudinal edges that are arranged in a curved state (e.g. rectangular fluid transport elements that are folded, curled or wrapped), fluid transport elements with curved longitudinal edges in a flat state, or fluid transport element s with curved longitudinal edges that are folded curled or wrapped and thus in a curved state).

In an embodiment of the testing device of the third aspect of the present invention, a width direction of the fluid transport element extends at an angle smaller than 90° with respect to the normal of the plane defined by the support structure. Thus, a longitudinal edge of the fluid transport element is arranged on the support structure so that the other longitudinal edge extends from the support structure. Also, the fluid transport element is curved, resulting in a shortest distance between two opposite longitudinal ends of the center line being shorter than the center line length in the planar state. The shortest distance is herein defined as a length amount indicative of a minimum distance amount between a proximal end of the fluid transport element (i.e. a section of the testing strip being in contact with or in the vicinity of the piercing element or the piercing element carrier) and a distal end of the fluid transport element at which, or close to which, the testing unit is arranged.

It is noted that a shortest distance between the longitudinal ends of the fluid transport element shorter than a center line length implies that the testing strip is curved, either in its planar state, or because the fluid transport element is arranged non-planar or both. Fluid transport elements wherein the shortest distance between the longitudinal ends is shorter than its center line length have an effective total extension or envelope of the fluid transport element that is smaller than the center line length in the planar state. This, in turn, allows for a size reduction of the testing device in comparison with a minimum size that the assembly would have in case the fluid transport elements were arranged in the planar state. If the fluid transport element is curved in a semi-circular shape, the shorter distance between the longitudinal ends may be shorter that the maximum outer dimension of the fluid transport element while the maximum outer dimension of the fluid transport element is still smaller than the center line.

This advantageous spatial arrangement of the fluid transport element in the testing device enables an improved usage of space within the testing device. It further enables a reduction of a total size of the testing device without a need to reduce the center line length of the fluid transport element. This, in turn, results in an improved applicability and offers an increased versatility.

By arranging the fluid transport element in a curved way and in a way in which the width direction extends at an angle smaller than 90° with respect to the normal of the plane defined by the support structure (i.e. the fluid transport element not being arranged parallel to the plane defined by the support structure), the size of the testing device can be reduced compared to typical testing device configurations, wherein the fluid transport element such as testing strips are usually directly arranged on a support structure in the planar state. Alternatively, longer fluid transport elements can be used when compared to known testing devices wherein the testing strips are arranged in the planar state onto the support structure.

In another embodiment, the testing device additionally or alternatively comprises at least one solution chamber containing a respective buffer solution, and flow control means configured to control a transfer of the buffer solution to the fluid transport element.

The at least one solution chamber is preferably arranged on the support structure. In a particular embodiment, the solution chamber is provided as a cavity in the support structure.

Some buffer solutions are advantageously chosen to enhance the transfer of the fluid sample to the testing unit. Other buffer solutions comprise a reagent that is configured to react with a particular analyte in a predetermined manner. In a particular embodiment comprising a plurality of solution chambers, different solutions chambers may contain different buffer solutions, which are individually transferred to one or more of the fluid transport element in accordance with particular requirements of the testing devices.

Solution chambers and buffer solutions such as those discussed above can be used in combination with any of the technical features described with respect to the previous embodiments.

In an embodiment of the testing device of the third aspect of the invention, the flow control means is configured to control a transfer of the buffer solution from the solution chamber to at least one of the fluid transport elements either before a fluid sample is transferred to the at least one fluid transport element, or while the fluid sample is being transferred to the at least one fluid transport element, or after the fluid sample has been transferred to the at least one fluid transport element, or any combination thereof.

Transferring the buffer solution to the fluid transport element before the fluid sample is received or transferred causes a wetting of the capillary bed or wick or of an absorbent material, which, in a particular embodiment, enhances an absorption capacity of the material.

Transferring the buffer solution to the fluid transport element while the fluid sample is being received or transferred increases the volume of the liquid present and the flow velocity of the fluid sample and thus reduces the time needed by the fluid sample to reach the testing unit.

Transferring the buffer solution to the fluid transport element after the fluid sample has been received or transferred is advantageously used in a particular embodiment to wash away the fluid sample towards the testing unit.

Any of the variants regarding the transfer of the buffer solution can be used in combination with any of the technical features discussed with regard to any of the previous embodiments of the testing device of the third aspect of the invention.

The flow control means may comprise, in an embodiment in accordance with the third aspect of the invention, a soluble material configured to be dissolved in the buffer solution at a predetermined dissolution rate and configured to enable a flow of the buffer solution away from the respective solution chamber after a predetermined time span. In another embodiment, the testing device comprises a reservoir containing a soluble material, for instance a pharmacologically inactive substance such as for example lactose monohydrate. This soluble material is configured to be dissolved when in contact with a predetermined fluid such as a body fluid. The dissolution of the soluble material is configured to bring in contact a piercing means with the solution chamber. The piercing means is configured to pierce the solution chamber and to allow a controlled flow of the buffer solution out of the solution chamber.

A particular embodiment of a testing device in accordance with the third aspect of the invention additionally or alternatively comprises hollow needles or catheters or microfluidic connection systems filled with the soluble material. The hollow needles or catheters are configured to pierce the solution chamber upon operation (e.g. by applying pressure, or by actuating the testing device in a predetermined manner). Once the solution chamber is pierced, the buffer solution enters in contact with the soluble material. Thus, by a proper choice of the soluble material, its amount, and the geometry of the flow control means and the solution chamber, a time span expanding between piercing the solution chamber and the buffer solution reaching the fluid transport element is controlled.

In another embodiment in accordance with the third aspect, which may also include any of the technical features described with respect to any of the preceding embodiments, the testing device further comprises a window section being at least partially transparent in a visible wavelength range and arranged to allow an optical inspection of the testing unit from outside the testing device.

In a particular embodiment, the window section is included in the peripheral support structure. In an embodiment wherein the testing device comprises a reflector element to allow an optical inspection of the testing unit from a direction substantially perpendicular to the plane defined by the support structure, the window section is advantageously arranged so that its projection onto the plane encloses at least a portion of the reflector element. In an alternative embodiment, the support structure of the testing device has a peripheral wall with in a direction substantially perpendicular to the plane, and the window section is arranged in said peripheral wall at peripheral wall positions that enable a direct optical inspection of the testing units. Yet another embodiment comprises a respective window section in an upper part of support structure and in the peripheral wall.

In a particular embodiment of the testing device of the third aspect of the invention, the window section is arranged in a recessed region of the upper part of the peripheral support structure, i.e. not on the peripheral wall. In this embodiment, the window section may comprise at least one lens, preferably at least a collimating lens. In an embodiment, the at least one lens is made of glass. In an alternative embodiment, the at least one lens is made of an acrylic compound. Another alternative embodiment may comprise other transparent materials known to the person skilled in the art. In yet another embodiment, the window section comprises an array of micro lenses.

Any of the variants discussed with respect to the window section may be combined with any of the technical features disclosed with regard to the previous embodiments of the testing device.

An embodiment of the testing unit wherein the window section is arranged in a recessed region of the upper part of the peripheral support structure offers an increased protection of the window section, by reducing the risk of damaging it, by, for instance, unintended scratching. Further, this embodiment is advantageously configured to be used in combination with a test-reading device comprising a photographic camera such as, for instance, a mobile phone device. In some test-reading devices, the camera protrudes from a backside plane of a housing thereof. By arranging the window section in a slightly recessed region, the mobile phone can be placed thereon, maximizing a contact surface between the back side of the test-reading device and the testing device, thus reducing the risk of an incorrect relative positioning. In an embodiment of the testing device of this invention, the peripheral support structure may also comprise a non-slip or anti-skid material configured to increase the friction of the testing device when in contact with other object such as a test-reading device.

The test-reading device may comprise a processor and a memory unit including a software causing the test-reading device to access the camera, analyze the image and interpret the state of the reacting material of the test portion according to a predetermined analysis template. The software may be configured to provide an output signal that comprises result data pertaining to the state of the reacting material. The output signal can then be directly sent to a user interface or, via a wired or wireless communication link to a centralized data processing center, or both.

In these cases, it is desirable to allow an entrance of light into the testing device to illuminate the testing unit during a reading phase of the state thereof. Since it is possible that the test-reading device blocks light paths from an exterior of the testing unit through the window section, an embodiment of the testing device preferably comprises further transparent or translucent sections of the peripheral support structure configured to allow light to penetrate inside the testing device.

Some particular test-reading devices further comprise a source of light such as a flash unit. Depending on the relative spatial arrangement of the camera and the source of light, light from the source of light can also enter the testing device through the window section. However, in order to provide a general solution, independently of a particular type of test-reading device, i.e. whether the test-reading device comprises a source of light or its relative arrangement with respect to the camera, an embodiment of the testing device comprises an additional window section at the peripheral wall. Additionally, or alternatively, an embodiment of the testing device further comprises light guiding means to guide the light from the additional window section at the peripheral wall to the testing units for illumination. For instance, in a particular embodiment of the testing unit, the peripheral wall serves as a light guide. In another embodiment of the testing device, the additional window section may additionally or alternatively comprise a transparent or translucid material, preferably glass or an acrylic compound. In a particular embodiment, the material is mated to enable an even distribution of light.

In another embodiment, the testing device is advantageously configured to read the test result and to transfer information pertaining to this test result to an external device. In an embodiment, the testing device further comprises an optical sensor, arranged and configured to detect light reflected from the at least one testing unit and to convert the detected light into an electrical signal representing an intensity and/or a color of the detected light. It further comprises a conversion unit for converting the electrical signal into digital data representing the intensity and/or the color of the detected light, and a transmitter unit for wirelessly transmitting the digital data to the external device.

The testing device's optical sensor is arranged and configured for detecting light reflected from the at least one testing unit, and for providing an electrical signal representing an intensity and/or a color of the detected light. The optical sensor can be, e.g., a single-pixel photodiode or a CMOS-sensor or a CCD-sensor. After converting detected light into an electrical signal, the electrical signal is provided to the conversion unit. The conversion unit is configured for converting the electrical signal into digital data representing the intensity and/or the color of the detected light. The conversion unit can be part of the optical sensor. The conversion unit can also be a separate component of the testing device. Alternatively, the conversion unit can be part of the transmitter unit. Preferably, the conversion unit is or comprises an analog-to-digital converter (ADC) for converting the electrical signal into digital data representing the intensity and/or the color of the detected light. The conversion unit can be configured for converting the electrical signal with 8-bit.

The conversion unit is operatively connected to the transmitter unit, e.g., via a data bus, for providing digital data to the transmitter unit.

The transmitter unit is configured for wirelessly transmitting the digital data; preferably, to an external receiving device using a predetermined wireless communication protocol such as Bluetooth, near-field communication (NFC) or Wi-Fi or RFID. In particular, the transmitter unit can be or can comprise a NFC-chip and a NFC-coil, or a radio-frequency identification (RFID)-tag or transponder, or a Wi-Fi-integrated circuit chip, or a Bluetooth integrated circuit chip.

A transmitter unit based on a technology such as NFC or RFID not requiring a permanent energy supply is preferred. The energy needed for powering such a transmitter unit is provided by a so-called initiator.

A transmitter unit that is configured for transmitting data via NFC or RFID, preferably, comprises one or more antennas functioning as a radio-frequency (RF) interface for transmitting electromagnetic signals representing digital data by means of electromagnetic induction to one or more further antennas of an external device. Antennas typically comprise one or more coils each having four or five windings.

The initiator can be the external device providing a carrier field that is modulated by the transmitter unit for transmitting digital data. Preferably, for powering the transmitter unit, the transmitter unit draws energy from the external device via the NFC or RFID link. Thus, in particular, in case the transmitter unit is NFC- or RFID-enabled, it is not necessary that the testing device itself comprises an energy storage unit, e.g., a battery, for powering the transmitter unit.

The optical sensor, the conversion unit and the transmitter unit can be arranged as separate components on the peripheral support structure together with any microfluidic components also comprised by the testing device. It is also possible that the optical sensor, the conversion unit and the transmitter unit are be fabricated using electronic packaging, e.g., 3D-packaging. Using 3D-packaging, thus, by stacking the components on top of each other, a compact three-dimensional integrated circuit can be designed. After 3D-packaging the integrated circuit comprising the optical sensor, the conversion unit and the transmitter unit can be mounted onto the peripheral support structure or attached to a cover unit. Alternatively, a chip comprising the optical sensor, the conversion unit and the transmitter can be fabricated using wafer-level packaging (WLP). The optical sensor, the conversion unit and the transmitter unit can also be put into a protective package for integration into the testing device.

In some embodiments, the optical sensor, the conversion unit and the transmitter unit are mounted on a circuit board that is attached as a module onto the peripheral support structure or to a cover unit. The circuit board can be flexible, e.g., a flexible substrate made of polyimide, e.g., Kapton, polyether ether ketone (PEEK), liquid-crystal polymer (LCP), or FR4. Also a rigid or semi-flex circuit board can be used as an alternative. In particular, the optical sensor, the conversion unit and the transmitter can be integrated onto a thin FR4 substrate. The circuit board can be a printed circuit board (PCB) which, preferably, is flexible, e.g., a FR4 PCB. Alternatively, the printed circuit board can be a rigid or semi-rigid printed circuit board.

Attaching at least one of the optical sensor, the conversion unit or the transmitter unit to a top or to a peripheral section of the support structure is of advantage since more space is left on a bottom section of the support structure, e.g., for arranging the components of the microfluidic system.

In particular, an antenna of the transmitter unit can be integrated in the testing device using in-mold manufacturing. In case the testing device has a cover unit attached to the support structure, thus, forming a closed housing, an antenna of the transmitter can be integrated into the housing, e.g., attached on the inside to the lid part of the cover unit by means of in-mold manufacturing. Alternatively, an antenna of the transmitter unit can be integrated into the same chip or in the same circuit as the remaining transmitter electronics, the optical sensor and the conversion unit. For instance, the antenna can be integrated in the PCB.

Preferably, in another embodiment, the testing device comprises an optical arrangement comprising one or more optical elements that are arranged and configured for directing light reflected from the at least one testing unit to the optical sensor and/or for directing light emitted from a light source to the at least one testing unit. An optical element can be a mirror or a lens or a waveguide. The optical elements serve for creating an optical path linking, e.g., the at least one testing unit to the optical sensor.

For example, a mirror can be used for directing light reflected from the testing unit and a lens can be employed for focusing the light reflected from the mirror onto the optical sensor. It is also possible that a waveguide is used for directing the light reflected from the at least one testing unit to the optical sensor. The waveguide can be shaped at its one end for focusing light onto the optical sensor.

An optical arrangement is of advantage, in particular, if the testing unit and the optical sensor are not aligned, i.e., the testing unit is not in the immediate field of view of the optical sensor.

An optical arrangement is also of particular advantage if a testing device comprises more than one testing units, e.g., three testing units. In this case, an optical element of the optical arrangement, preferably, is configured and arranged for directing light reflected from anyone of the three testing units to the optical sensor. An accordingly configured optical element can comprise three mirror surfaces that are inclined to each other in a way that light reflected from anyone of the testing units is directed to the optical sensor upon reflection on one of the three inclined mirror surfaces. Thus, in general, an optical element can comprise a plurality of reflecting surfaces, e.g., a plurality of facets, that are configured and arranged for directing light reflected from one or more testing units that are arranged along the length of one fluid transport element to the optical sensor. An external receiving device can receive the digital data from the transmitter unit wherein the digital data representing light intensity and/or color of the light reflected from one or more of the plurality of testing units.

In general, using an optical arrangement allows to more freely arrange the one or more fluid transport elements and the optical sensor in the testing device, e.g., on the bottom part of the peripheral support structure since light paths can be created connecting testing unit and optical sensor. Such light paths can have an angle or can be curved, e.g., when using waveguides.

According to a fourth aspect of the present invention, a method for actuating a hand-operated actuator mechanism for driving an external piercing element is disclosed, the method comprises:
- applying a predetermined pushing finger force on a pushing member, thereby breaking a first breakable connection element connecting a mounting element to a trigger element;
- moving a piercing element carrier connected via a second breakable connection element to the trigger element from an initial position wherein the first and the second breakable connection elements are unbroken, to a stop position wherein an abutment (116) stops a movement of the piercing element carrier.

In a particular embodiment, the method further comprises breaking the second breakable connection element when the piercing element carrier reaches the stop position and acting on the piercing element carrier for returning the piercing element carrier from the stop position in the direction of the initial position.

In yet another embodiment breaking the first breakable connection element requires applying a pushing finger force of 20-100 N.

According to a fifth aspect of the present invention, a method for operating a testing device is disclosed. The method comprises performing the steps of any of the methods of the fourth aspect, transporting a fluid via a fluid transport element from a piercing element to at least one testing unit and reacting a respective reacting material in a predetermined manner to a pre-specified analyte or property of the fluid.

In an embodiment, the method of the fifth aspect also comprises detecting light reflected from at least one testing unit, converting the light into an electrical signal representing an intensity and/or a color of the detected light, converting the electrical signal into digital data representing the intensity and/or the color of the detected light and wirelessly transmitting the digital data, preferably using a near-field communication link.

It shall be understood that the a hand-operated actuator mechanism of claim 1, the piercing device of claim 5, the testing device of claim 10 and the methods for operating the actuator mechanism and the testing device of claims 18 and 21 respectively, have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiment described hereinafter.
Fig. 1A shows a schematic diagram of an embodiment of a hand-operated actuator mechanism in a non-actuated state.
Fig. 1B shows a schematic diagram of the embodiment of the hand-operated actuator mechanism of Fig. 1A in an actuated state.
Fig. 2A shows a schematic diagram of another embodiment of a hand-operated actuator mechanism in a non-actuated state.
Fig. 2B shows a schematic diagram of the embodiment of the hand-operated actuator mechanism of Fig. 2A in an actuated state.
Fig. 2C shows a schematic diagram of the embodiment of the hand-operated actuator mechanism of Figs. 2A and 2B in a final state.
Fig. 3 shows a schematic diagram of another embodiment of a hand-operated actuator mechanism.
Fig. 4A shows a schematic diagram of another embodiment of a hand-operated actuator mechanism in a non-actuated state.
Fig. 4B shows a schematic diagram of the embodiment of the hand-operated actuator mechanism of Fig. 3A in an actuated state.
Fig. 4C shows a schematic diagram of the embodiment of the hand-operated actuator mechanism of Figs. 3A and 3B in a final state.
Fig. 5A shows a schematic diagram of an embodiment of a piercing device with a hand-operated actuator mechanism in a non-actuated state.
Fig. 5B shows a schematic diagram of the embodiment of the piercing device of Fig.5A with the hand-operated actuator mechanism in an actuated state.
Fig. 5C shows a schematic diagram of the embodiment of the piercing device of Figs.5A and 5B with the hand-operated actuator mechanism in a final state.
Fig. 6A shows a schematic diagram of another embodiment of a hand-operated actuator mechanism in a non-actuated state.
Fig. 6B shows a schematic diagram of a breakable connection element.
Fig. 7 shows a schematic diagram of an embodiment of a testing device.
Fig. 8A and 8B show a set of fluid transport elements in a planar state and in a curved state respectively.
Fig. 9 shows a schematic diagram of another embodiment of a testing device.
Fig. 10 shows a schematic diagram of another embodiment of a testing device.
Fig. 11 shows a flow diagram of an embodiment of a method for operating a hand-held forced-controlled actuator mechanism.
Fig. 12 shows a flow diagram of an embodiment of a method for operating a testing device.

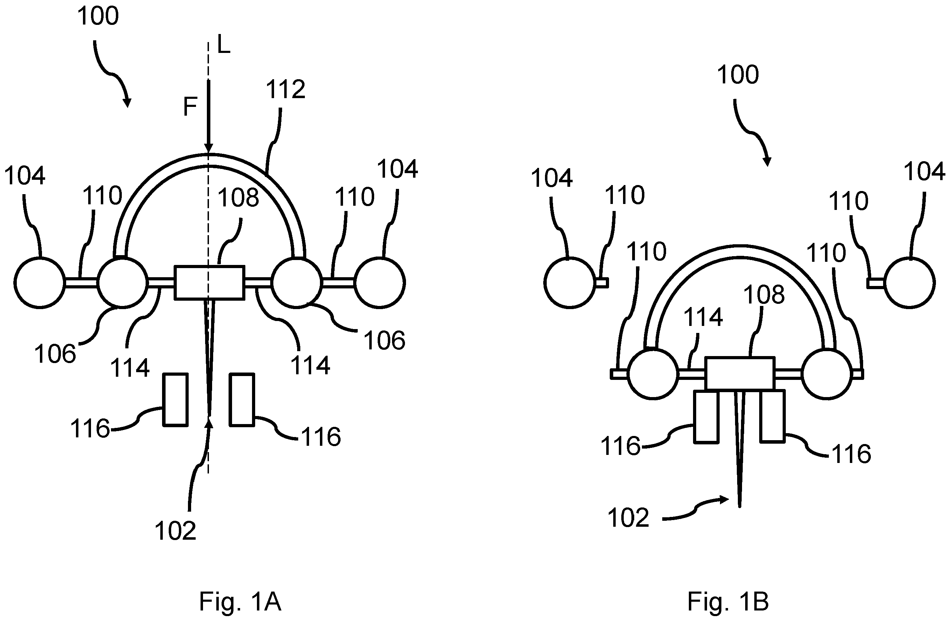

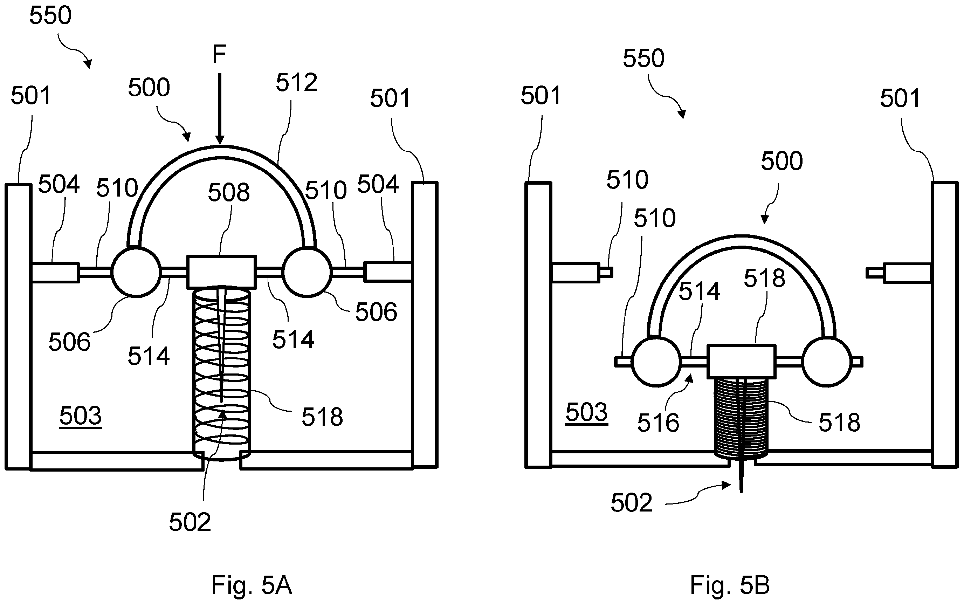

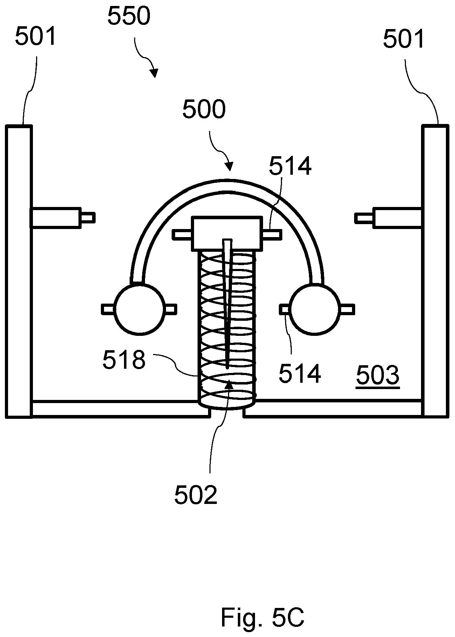

Fig. 1A shows a schematic diagram of an embodiment of a hand-operated actuator mechanism 100 in a non-actuated state. Fig. 1B shows a schematic diagram of the same the hand-operated actuator mechanism 100 of Fig. 1A in an actuated state. The hand-operated actuator mechanism 100 is particularly suitable for driving an external piercing element 102. The actuator mechanism 100 is suitable for integration in a piercing device, as it is described with reference to Figs. 5A, 5B and 5C. The actuator mechanism comprises a mounting element 104, a trigger element 106 and a piercing element carrier 108. The external piercing element 102 is operatively connected to the piercing element carrier 108.

The trigger element 106 is connected to the mounting element 104 via a first breakable connection elements 110. The trigger element 106 further comprises or is connected to a pushing member 112 that can be pushed with a human finger. In the actuator mechanism, the first breakable connection elements 110 are configured to break if a pushing finger force F that pushes the pushing member 112, particularly in a longitudinal direction L, exceeds a predetermined threshold. In the actuator mechanism 100, the piercing element carrier 108 is connected to the trigger element 106 via a second breakable connection element 114.

Once the first breakable connection elements 110 are broken, the piercing element carrier 108 can move from an initial position to a stop position. The initial position is shown in Fig. 1A as an non-actuated state, wherein the first and the second breakable connection elements 110, 114 are unbroken. The stop position is shown in Fig, 1B as an actuated state, wherein an abutment 116 stops a movement of the piercing element carrier 108, in the longitudinal direction.

In the exemplary actuator mechanism 100 of Figs. 1A and 1B, the positions of the abutment and the mounting element are fixed and thus constant relative to each other. Once the predetermined pushing finger force F is applied on the pushing member 112, the first breakable connection elements 110 break and the trigger element and the piercing element carrier are movable, relative to the mounting element, between the initial and the stop position.

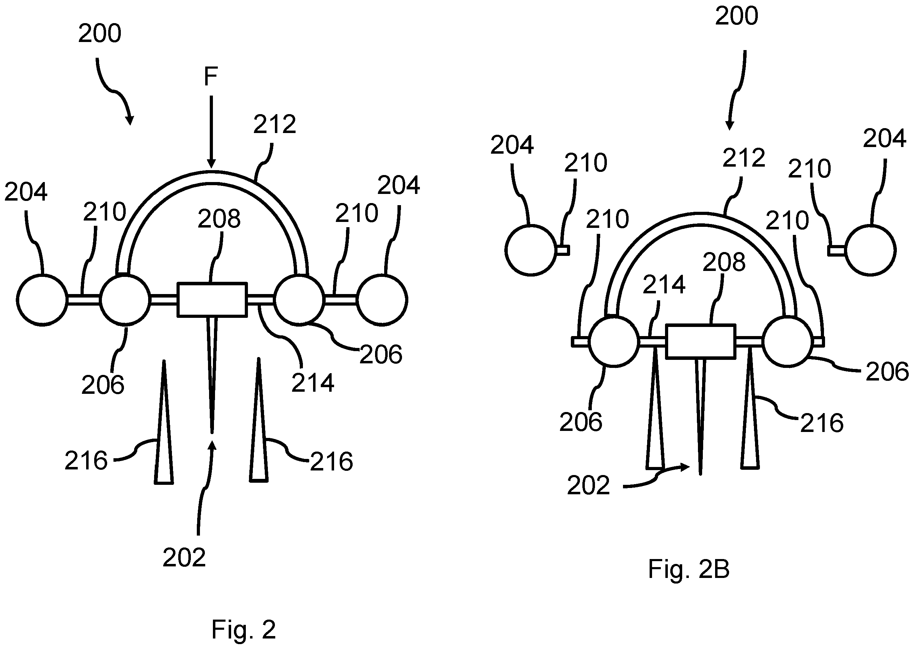

Fig. 2A shows a schematic diagram of another embodiment of a hand-operated actuator mechanism 200 in a non-actuated state. Fig. 2B is a schematic diagram of the hand-operated actuator mechanism 200 of Fig. 2A in an actuated state. The following discussion focuses on those features that are different between the actuator mechanisms 100 and 200. The technical features being similar or identical or having similar or identical functions are referred to using the same numerals, except for the first digit, that is "1" for the actuator mechanism 100 of Figs. 1A and 1B and "2" for the actuator mechanism 100 of Figs. 2A, and 2B

In the actuator mechanism 200, the second breakable connection elements 214 are configured to break when the piercing element carrier 208 reaches the stop position shown in Fig. 2B. In this particular example, the abutment 216 is advantageously designed and arranged to break the second breakable connection elements 214 when the piercing element carrier 208 and/or the second breakable connection elements 214 moving together with the trigger element hit the abutment 216. Breaking of the second breakable connection elements 214 decouples the piercing element-carrier 208 from the trigger element 206. This ensures that the actuator mechanisms can only be used once.

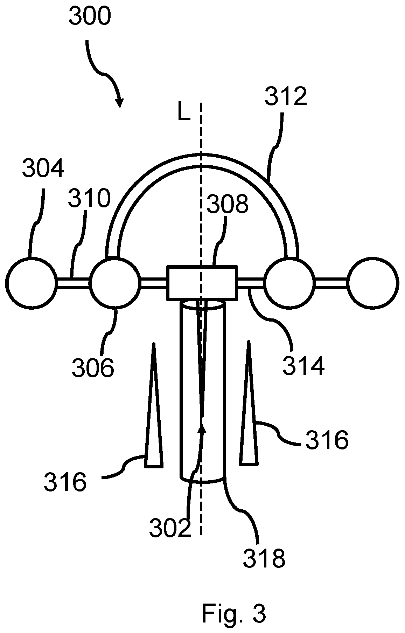

Fig. 3 shows a schematic diagram of another embodiment of a hand-operated actuator mechanism 300 in a non-actuated state. The following discussion focuses on those features that are different between the actuator mechanism 300 of Fig. 3 and the actuator mechanisms 100 and 200. The technical features being similar or identical or having similar or identical functions are referred to using the same numerals, except for the first digit, that is "3" for the actuator mechanism 300 of Fig. 3, and "1" and "2" for the actuator mechanism 100 and 200 of Figs. 1A, 1B and Figs. 2A, 2B respectively.

The actuator mechanism 300 of Fig. 3 further comprises an elastic element 318 that is arranged to act on the piercing element carrier 308 for returning the piercing element carrier form the stop position in the direction of the initial position. In this particular actuator mechanism 300, the elastic element is a helical coil spring that is compressed when the piercing element carrier 308 is pushed in the longitudinal direction by the applied pushing finger force once this force exceeds a predetermined trigger value for breaking the first breakable connection element 340. As in the embodiment of figure 2, the abutment 316 is configured to break the second breakable connection elements 314 and decouple the trigger element 306 from the piercing element carrier 308 once the second breakable connection and/or the piercing element carrier 308 hit the abutment 316. The spring force of the compressed elastic element 318 induces a movement of the piercing carrier element 308 from the stop position in the direction of the initial position.

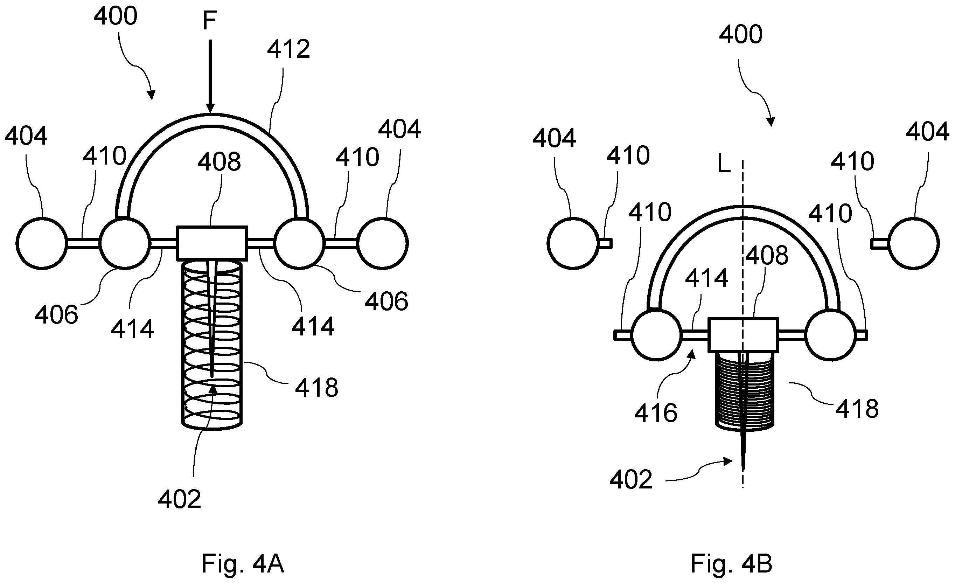

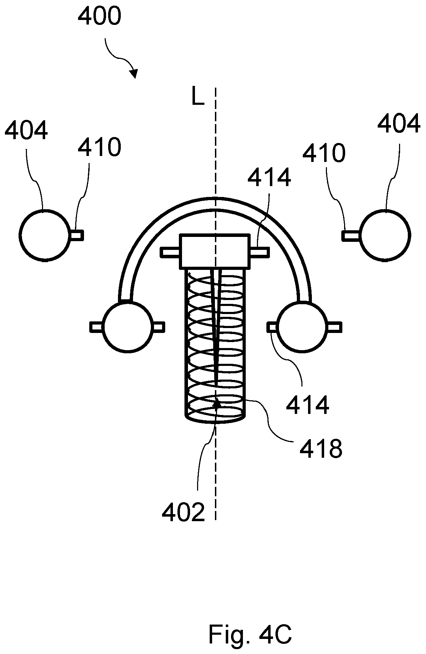

An alternative embodiment of an actuator mechanism 400 is shown in an initial, non-actuated state, in an intermediate actuated state and in a retracted final state in Figs. 4A, 4B and 4C respectively. The following discussion focuses on those features that are different between the actuator mechanism 400 of Figs. 4A, 4B and 4C, and the actuator mechanisms 100, 200 and 300 of Figs 1 to 3. The technical features being similar or identical or having similar or identical functions are referred to using the same numerals, except for the first digit, that is "4" for the actuator mechanism 400, and "1", "2" and "3" for the actuator mechanism 100, 200 and 300 respectively.

In the actuator mechanism 400 of Figs. 4A, 4B and 4C, the fully compressed elastic element 418 acts as an abutment 416 that is arranged and configured to stop the movement of the piercing element carrier 408. Once the first breakable element 410 has been broken upon application of the predetermined pushing finger force F, the elastic element 418 is compressed. Once fully compressed the elastic element 418 stops the movement of the piercing element carrier 408 and thus acts as an abutment 416. Thus, in this particular actuator mechanism, the functionality of both the abutment 416 and the elastic element 418 are combined in the elastic element 418 arranged at a particularly advantageous position.

In the example shown in Fig. 4A, 4B and 4C, the application of the predetermined pushing finger force F on the pushing member 412 causes the first breakable connection elements 410 to break, causing an accelerated movement of the trigger element 406 and compressing the elastic element 418, in this case a coil spring, in particular a helical coil spring. The elastic element 418 is arranged and configured to stop the movement of the piercing element carrier 408 at the stop position shown in Fig. 4B. An upper edge of the spring coil is in contact with the piercing element carrier 408 and the compressed elastic element 418 (i.e. the helical spring 418 in its fully compacted, solid state) causes a counter force to the finger force driving the trigger element 406. Once the counterforce exceeds the load the second breakable connection elements 414 are designed to withstand, the second breakable elements 414 break. The counterforce can for example exceed the load the second breakable connection elements 414 are designed to withstand when the helical spring is fully compressed orwhen the spring force due to compression of the spring is large enough. Once the second breakable elements 414 are broken, the trigger element 406 is decoupled from the piercing element carrier 408, and the spring force of the elastic element acts on the piercing element carrier 408 inducing a retraction movement of the piercing carrier element 408 towards the initial position.

In particular, in any of the embodiments of the actuator mechanism described above, it is preferred that the first breakable connection is adapted to be broken upon exertion of a pushing finger force of 20-100 N on the pushing member. More preferably, the pushing finger force is between 35 and 45 N

Figs. 5A, 5B and 5C are schematic diagrams of an embodiment of a piercing device 550 with a hand-operated actuator mechanism 500 in an initial non-actuated state, in an intermediate actuated state and in a final retracted state respectively. The actuator mechanism 500 of this particular piercing device 550 shares the same features as the actuation mechanism 400 of Figs. 4A, 4B and 4C. The technical features are thus referred to using the same numerals except for the first digit, which is "5" for the features of the actuator mechanism 500 of and "4" for the features of actuator mechanism 400. The piercing device comprises a peripheral support structure 501 that forms an inner volume 503. The peripheral support structure has a base section forming a support plane and a peripheral wall, substantially perpendicular to the base section and preferably of a cylindrical shape. The piercing device 550 also includes a hand-operated actuator mechanism 500 such that the mounting element 504 is connected to the peripheral support structure 501, particularly to the peripheral wall.

In an exemplary piercing device, the piercing element carrier comprises attaching means for attaching an external piercing element, such as a lancet, a needle, a hollow needle, a catheter or any other suitable piercing element. In another exemplary piercing device, such as piercing device 550, the piercing element 502 is an integral part of the piercing device and is connected to the piercing carrier element.

Preferably, the piercing element 502 is dimensioned and arranged:
a) to remain within the inner volume 503 at the initial position shown in Fig. 5A;
b) to protrude from the inner volume 503 at the stop position shown in Fig. 5B, after the application of the predetermined pushing finger force F has caused the first breakable connection element 504 to break, thereby decoupling the mounting element 504 from the trigger element 506 and;
c) to return to the inner volume at the retracted final position shown in Fig. 5C, the retraction movement being caused by the stored elastic energy of the elastic element transferred to the piercing element carrier once the second breakable element have been broken.

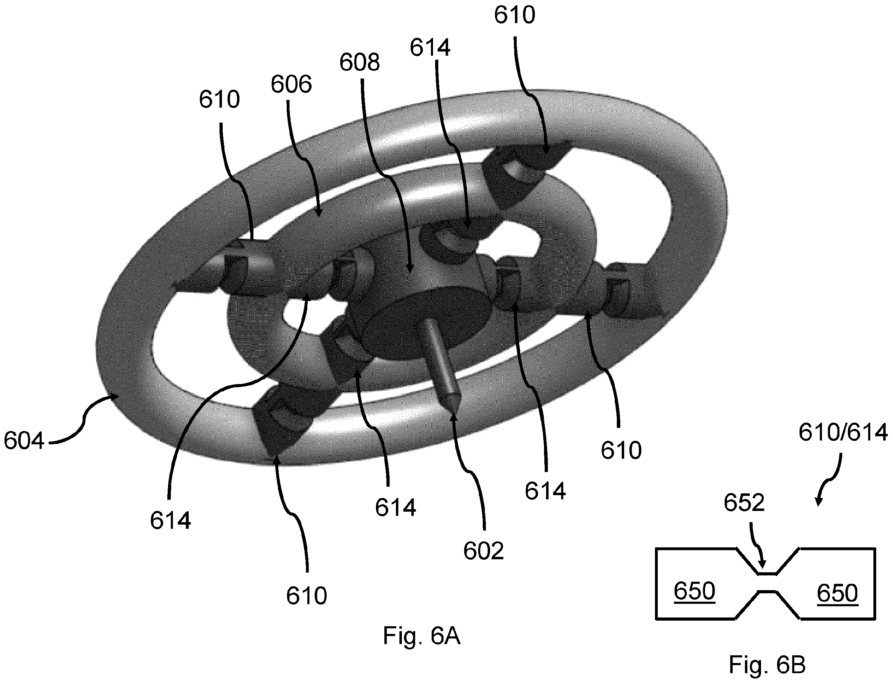

Fig. 6A shows a schematic diagram of another embodiment of a hand-operated actuator mechanism 600 in a non-actuated state. The mounting element 604 has a toroidal shape and is configured to be attached or connected to the peripheral support structure of the piercing device so as not to move relative to the peripheral support structure independently of the current state of the piercing device. The trigger element 606 is arranged concentrically to the mounting element 604, at a distance closer to a center point than the mounting element 604. The actuator mechanism comprises a piercing element carrier 608, to which an external piercing element 602 is attached. The piercing element carrier 608 is located at a central position of the actuator mechanism 600. A plurality of first breakable connection elements 610 connect the mounting element 604 to the trigger element 606. Also, a plurality of second breakable connection elements 614 connect the trigger element 606 to the piercing element carrier 608. In this particular actuator mechanism 600 there are four equally spaced first breakable connection elements 610 and four equally spaced second breakable connection elements 614. Other actuator mechanisms include a different number of first and/or second breakable connection elements.

A schematic diagram of an exemplary first and/or second breakable connection element is shown in Fig. 6B. The connection element extends between the two elements it connects i.e. the mounting element to the trigger element, and the trigger element to the piercing element carrier respectively along a linking direction. The cross section along the linking direction is shown in Fig 6B. At the two ends 650, the connection element has a larger girth than in the middle section, where a recess 652 is located. Applying the pushing finger force or the breaking force causes the first and the second breakable connection element to break at the recessed region. A proper dimensioning of this region and a suitable choice of the materials allow for producing breakable connection elements that break upon exertion of a pre-specified force amount. Preferably, the pushing member is configured to apply force on the recessed region of the first breakable connection element and the abutment or any other breaking element is configured to apply force to the recessed region of the second breakable connection elements.

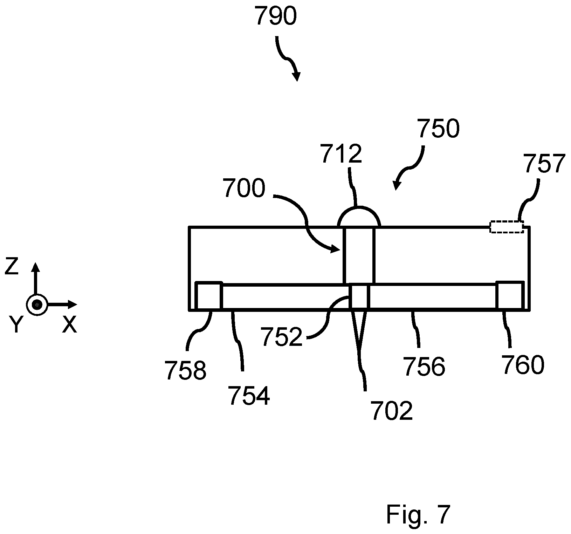

Fig. 7 shown a schematic diagram of an embodiment of a testing device 790. The testing device comprises a piercing device 750, such as the piercing device described with reference to Figs. 5A to 5C. The piercing device comprises an actuator mechanism 700 which controls a piercing movement of a piercing element 702 based on an applied pushing finger force on a pushing member 712. The actuator mechanism may also be configured to control a retraction movement of the piercing element, for example as explained with reference to Figs 4B and 4C. The testing device also comprises a fluid sample receiving unit 752 that is suitable for receiving a fluid sample for being tested. The fluid sample receiving is fluidly connected to a respective input end of two fluid transport elements 754, 756. The fluid transport element is arranged and configured to transport a fluid away from the input end. Suitable fluid transport elements include, but are not limited to capillary beds, such as capillary wicks, and microfluidic systems. The testing device also includes two testing units 758, 760 in fluidic communication with a respective fluid transport element. The testing units 758, 760 comprising a respective reacting material configured to react in a predetermined manner to a pre-specified analyte or property of the fluid.

The testing units 758, 760 may include a respective conjugate pad that comprises an immobilized conjugate material. The conjugate pad is configured to release the immobilized conjugate material upon contact with the liquid sample. The conjugate material is contained in the conjugate pads, i.e. as colloidal gold, or colored, fluorescent or paramagnetic monodisperse latex particles that have been conjugated to one specific biological component expected to be identified in the liquid sample. This biological component is in some testing devices an antigen and in other testing devices an antibody. The testing unit may include a test line and a control line forming a so-called reaction matrix. The fluid sample, received through the fluid sample receiving unit is transported by capillary action from the fluid sample receiving unit along the capillary wick. At the conjugated pad, the liquid sample releases the conjugate material and a combination of both is further transported towards an absorbent pad located at a distal end of the fluid transport element, such as a testing strip, opposite to a proximal end whereto the fluid sample receiving unit is connected. The absorbent pad is typically configured to act as a sink for the liquid sample, maintaining a flow of the liquid over the capillary wick and preventing a flow of the liquid sample back to or towards the fluid sample receiving unit.

The testing device 790 optionally comprises a window section 757 being at least partially transparent in a visible wavelength range and arranged to allow an optical inspection of the testing unit 760 from outside the testing device 790.

Other testing devices (not shown) comprise a different number of fluid transport elements. Other testing devices (not shown) comprise one or more fluid transport elements having a plurality of testing units.

In testing devices using as a fluid transport element a capillary bed or wick configured to transport a fluid by capillarity effect, the fluid transport element has, in a planar state, a center line length, a width and a thickness that is shorter than the center line length and the width. In order to reduce the size of the testing device, the fluid transport elements of some testing devices are arranged so that a width direction of the fluid transport element extends at an angle smaller than 90° with respect to a normal of a support plane defined by the peripheral support structure. Further, the fluid transport element is curved, resulting in a shortest distance between two opposite longitudinal ends of the fluid transport element being shorter than the center line length in the planar state.

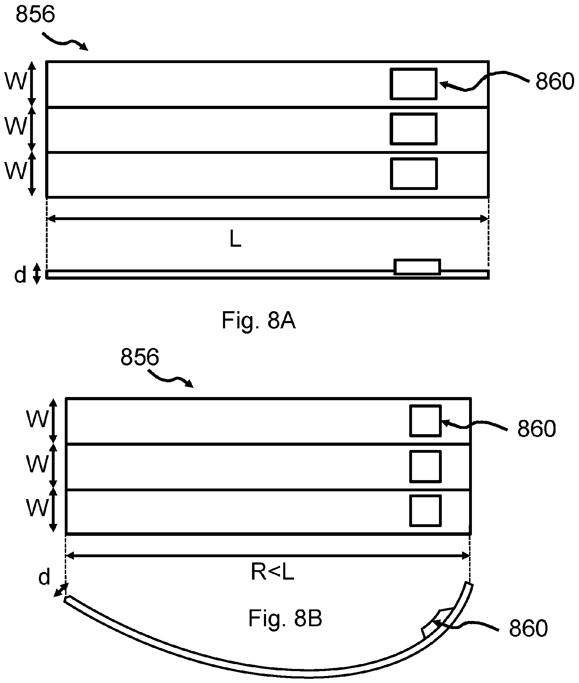

This is shown in Figs 8A and 8B, where the geometry of an exemplary set of fluid transport elements 856 in the form of testing strips, preferably comprising a capillary wick is described. In Fig. 2A, three testing strips form a set of testing strips. Each individual testing strip has a respective testing unit 860. Each testing strip is presented in Fig. 8A in a planar state and has a testing strip center line length L in a longitudinal direction, a testing strip width W in a width direction perpendicular to the longitudinal direction and a testing strip thickness d, in a thickness direction perpendicular to both the longitudinal direction and the width direction. The testing strip thickness d is shorter, i.e., has a smaller extension than the testing strip center line length L and the testing strip width W. Fig. 2B shows the same set of three testing strips 856 in a curved state in which a shortest distance between two opposite longitudinal ends of the testing strip center line, or in other words, an effective extension or envelop R of the testing strip, is shorter than the testing strip center line length L in the planar state shown in Fig. 2A. In this particular example, the shortest distance between the two opposite longitudinal ends of the testing strip corresponds to the effective extension R. In another exemplary configuration (not shown) wherein the testing strip is bent in e.g. a circular shape, the shortest distance between the two opposite longitudinal ends vanishes, whereas the effective extension corresponds to the diameter of the formed circle, which is π/*L*. In any case, the shortest distance and the effective extension are shorter than the testing strip center line length.

The fluid transport element, for instance the testing strip having a capillary wick, is advantageously arranged in the testing device 790 such that a width direction extends in the Z-direction, as indicated on Fig. 7. The support plane is that section of the peripheral support structure that lies in the plane XY, as indicated in Fig. 7. The peripheral walls of the peripheral support structure also extends substantially along the Z direction.

Preferably, the peripheral support structure has a flat or planar geometry that defines the support plane. In an alternative testing device, however, the support structure is not flat, but an outer perimeter of the support structure defines the plane. In yet another alternative testing device, neither the support structure nor the outer perimeter directly defines a plane and the plane is defined by averaging the spatial position of at least a part of the support structure or of the outer perimeter

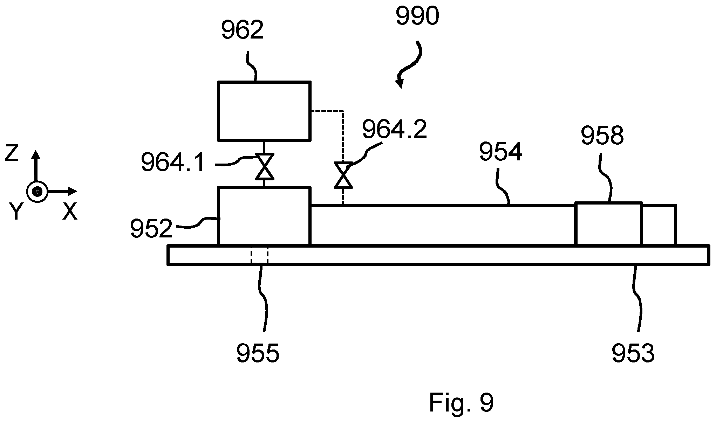

Fig. 9 shows a schematic representation of a testing device 990. For the sake of clarity, the features related to the actuator mechanism are not shown in the figure. The testing device 990 comprises a fluid sample receiving unit 952 arranged on a support structure that defines a support plane XY. The support structure 953 has an opening 955 for receiving the fluid sample. The testing device 990 further comprises a solution chamber 962 containing a buffer solution, and flow control means 964.1 configured to control a transfer of the buffer solution to the fluid sample receiving unit 952. Alternatively, or additionally, some testing devices include flow control means 964.2 that control a transfer of the buffer solution directly to the fluid transport element 954 (as indicated by the dashed-line). Some testing devices include a plurality of solution chambers and control flow means that control a respective transfer of the respective solution (which can be identical or different or a combination thereof) to the fluid sample receiving unit or to one or more of the fluid transport element, particularly testing strips preferably comprising a capillary wick. Buffer solutions are advantageously chosen to enhance a transport of the fluid sample along the capillary wick of the testing strips.

The flow control means 964.1, 964.2 is configured to control a transfer of the buffer solution from the solution chamber to the at least one fluid transport element or/and to the fluid sample receiving unit either:
- before a fluid sample is transferred to the at least one fluid transport element or/and to the fluid sample receiving unit; or
- while the fluid sample is being transferred to the at least one fluid transport element or/and to the fluid sample receiving unit; or
- after the fluid sample has been transferred to the at least one fluid transport element or/and to the fluid sample receiving unit; or
- any combination thereof.

Transferring the buffer solution to the fluid sample receiving unit or to the fluid transport element before the fluid sample is received or transferred respectively causes a wetting of the capillary wick or the absorbent material that in a particular embodiment enhances an absorption capacity.

Transferring the buffer solution to the fluid sample receiving unit or to the fluid transport element while the liquid sample is being received or transferred increases the volume of the liquid present and the flow velocity of the liquid sample and thus reduces the time needed by the liquid sample to reach the test portion of the testing strip.

Transferring the buffer solution to the fluid sample receiving unit or to the fluid transport element after the liquid sample is received or transferred is advantageously used in particular embodiment to wash away the liquid sample towards the test portion.

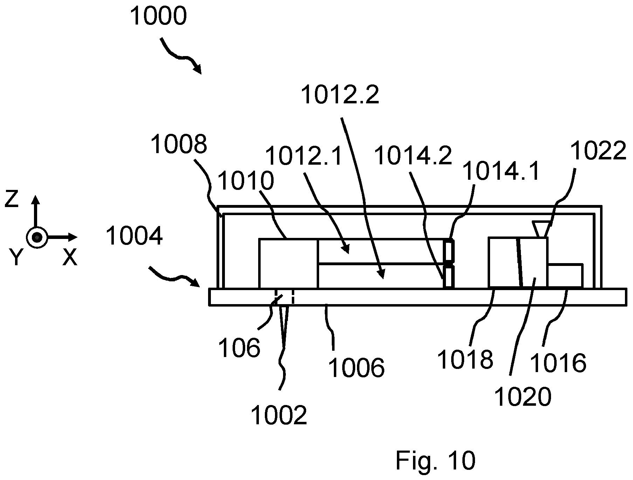

Fig. 10 shows a schematic diagram of another embodiment of a testing device 1000. As in the case of testing device 990 of Fig. 9, the actuator mechanism is not shown explicitly for the sake of clarity. The testing device 1000 comprises a piercing element 1002, such as a lancet. Other suitable piercing elements include, but are not limited to, needles, hollow needles, cannulas or catheters. Other testing devices (not shown) do not comprise a piercing element. The testing device 1000 includes a peripheral support structure 1004 including a support structure 1006 defining a support plane XY and a cover unit 1008 having a peripheral wall . The lancet is a particular and non-limiting example of piercing element and is connected to a fluid sample receiving unit 1010. The testing device further comprises a two fluid transport elements, in particular two testing strips 1012.1 and 1012.2 having a capillary wick, and each comprising a respective testing unit 1014.1 and 1014.2.

The testing device 1000 further comprises an optical sensor 1018 that is configured for detecting impeding light that is reflected by the testing units 1014.1 and 1014.2, and for converting the detected light into an electrical signal representing the intensity and/or the color of the impeding light. The optical sensor 1018 is connected to a conversion unit 1020. The conversion unit 1020 is configured for converting an electrical signal into digital data representing an intensity and/or a color of the detected light. The conversion unit 1020 is, in a particular testing device, an analog-to-digital converter and is comprised by the optical sensor 1018. The testing device 1000 also comprises a power management unit 1016 comprising voltage stabilizing circuitry.

Alternatively, the conversion unit 1020 can be a separate component that is arranged on the support structure 1006 and operatively connected to the optical sensor 1018. For example, the optical sensor 111, and a transmitter unit 1022 having an RF-interface can be arranged on the support structure 1006. Light reflected from the testing units 1014.1 and 1014.2 can be directed to the optical sensor using one or more mirrors also arranged on the support structure 1006 (not shown). Using mirrors, a light path can be created linking the testing units 1014.1 and 1014.2 and the optical sensor 1018. It is also possible that the optical sensor 1018, the transmitter unit 1022 having an RF-interface, and eventually also the power management unit 1016 are arranged on a circuit board, e.g., a flexible PCB. The circuit board can be arranged on the support structure 1006. Using optical elements such as mirrors a light path can be created from the testing units 1014.1 and 1014.2to the optical sensor 1018 that is arranged on the circuit board. The circuit board can also be attached to the inner surface of the cover unit 1008, the inner surface facing the support structure 1006. Preferably, the optical sensor 1018 is arranged such that if the circuit board is attached to the inner surface of the cover unit 1006, the optical sensor likewise faces the support structure. Since the testing units 1014.1 and 1014.2 face the sidewalls of the testing device 1000, preferably, an optical element is arranged and configured to redirect light reflected from testing units 1014.1 and 1014.2 about 90° towards the optical sensor 111. Further comprised can be one or more light sources, e.g., LEDs, that are arranged and configured for illuminating testing units 1014.1 and 1014.2. The one or more light sources can be arranged on the support structure 1006, or on a circuit board, or directly to the inner surface of the cover unit 1008.

The transmitter unit 1022 is connected to the power management unit 10016 and to the conversion unit 1020. The transmitter unit 1022 is configured to wirelessly transmit digital data representing the intensity and/or color of the detected light, e.g., in accordance with a predetermined wireless communication protocol. Preferably, in the testing device 1000, the transmitter unit 1022 is configured to transmit the digital data via a near-field communication link.

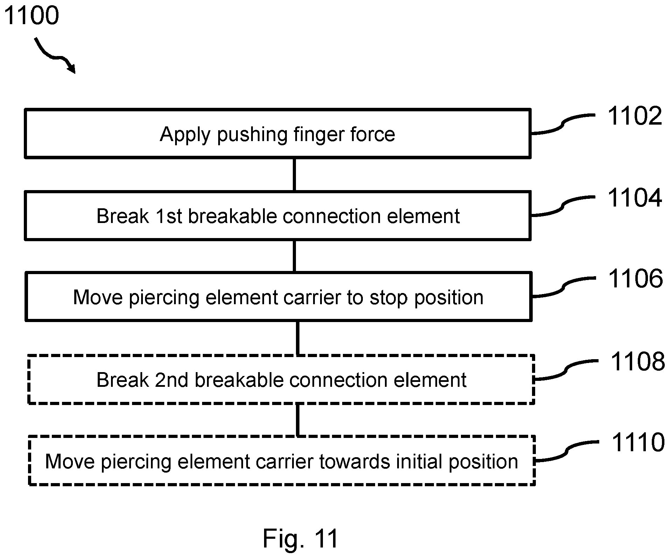

Fig. 11 shows a flow diagram of an embodiment of a method 1100 for actuating a hand-operated actuator mechanism for driving an external piercing element. The method comprises, in a step 1102 applying a predetermined pushing finger force on a pushing member, thereby breaking, in a step 1104, a first breakable connection element connecting a mounting element to a trigger element. The method 1100 also includes, in a step 1106, moving a piercing element carrier connected via a second breakable connection element to the trigger element from an initial position wherein the first and the second breakable connection elements are unbroken, to a stop position wherein an abutment stops a movement of the piercing element carrier.

A particular variant of the method 1100 also includes, in a step 1108, breaking the second breakable connection element when the piercing element carrier reaches the stop position, and, in a step 1110, acting on the piercing element carrier for returning the piercing element carrier from the stop position in the direction of the initial position. Preferably, breaking the first breakable connection element requires applying a pushing finger force of 20-100 N.

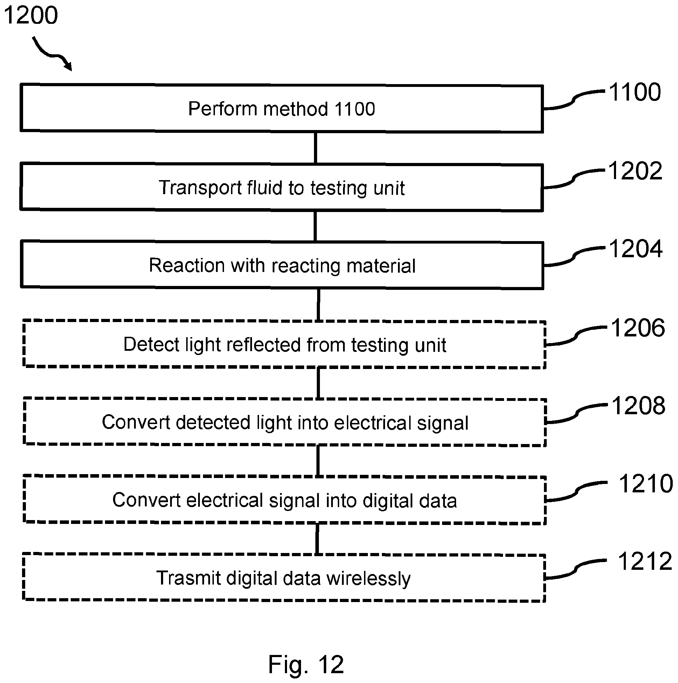

Fig. 12 shows a flow diagram of an embodiment of a method 1200 for operating a testing device. The method comprises performing the steps of the method 1100 of Fig. 11. The method also comprises, in a step 1202, transporting a fluid via a fluid transport element from a piercing element to at least one testing unit. The method also comprises, in a step 1204, reacting a respective reacting material in a predetermined manner to a pre-specified analyte or property of the fluid.

A particular variant of the method 1200 also includes, in a step 1206, detecting light reflected from at least one testing unit, in a step 1208, converting the light into an electrical signal representing an intensity and/or a color of the detected light, in a step 1210, converting the electrical signal into digital data representing the intensity and/or the color of the detected light, and in a step 1212 wirelessly transmitting the digital data, preferably via a near-field communication link.

In summary, the invention is related to a hand-operated actuator mechanism for driving a piercing element with increased safety. The actuator mechanism comprises a mounting element connected to a trigger element by a first breakable connection element. It also comprises a piercing element carrier connected to the trigger element by a second breakable connection element. The trigger element further comprises a pushing member that can be pushed with a human finger. The first breakable connection element is configured to be broken by means of a predetermined pushing finger force pushing the pushing member. The piercing element carrier being movable between an initial position wherein the first and the second breakable connection elements are unbroken, and a stop position wherein an abutment stops a movement of the piercing element carrier.

In the claims, the word "comprising" does not exclude other elements or steps, and the indefinite article "a" or "an" does not exclude a plurality.

A single unit may fulfill the functions of several items recited in the claims. The mere fact that certain measures are recited in mutually different dependent claims does not indicate that a combination of these measures cannot be used to advantage.

## Claims

1. A hand-operated actuator mechanism (100) for driving an external piercing element (102) with a predefined force, the actuator mechanism (100) comprising
- a mounting element (104);
- a trigger element (106);
- a piercing element carrier (108) for an external piercing element, wherein
- the trigger element (106) is connected to the mounting element (104) via a first breakable connection element (110) that is designed to break when a predefined force is applied to the trigger element, said trigger element (106) further comprising or being connected to a pushing member (112) that can be pushed with a human finger and that is arranged to transfer a finger force via the trigger element to the first breakable connection element so as to cause breaking of the first breakable connection element when a pushing finger force (F) pushing said pushing member (112) exceeds a predetermined threshold; and wherein
- the piercing element carrier (108) is connected to the trigger element (106) via a second breakable connection element (114), said piercing element carrier being movable between an initial position wherein the first and the second breakable connection elements (110, 114) are unbroken, and a stop position wherein an abutment (116) stops a movement of the piercing element carrier (108).

2. The actuator mechanism (200) of claim 1, wherein the second breakable connection element (214) is configured to be broken when the piercing element carrier (208) reaches the stop position.

3. The actuator mechanism (300) of claim 1 or 2, further comprising an elastic element (318) that is arranged to act on the piercing element carrier (308) for returning the piercing element carrier form the stop position in the direction of the initial position.

4. The actuator mechanism (400) of claim 3, wherein the abutment (416) is the elastic element (418) in a stressed or compressed state.

5. The actuator mechanism according to at least one of the preceding claims, wherein the first breakable connection is adapted to be broken upon exertion of a pushing finger force of 20-100 N on the pushing member.

6. A piercing device (550) comprising:
- a peripheral support structure (501) forming an inner volume (503);
- a hand-operated actuator mechanism (500) in accordance with any of the preceding claims; wherein the mounting element is connected to the peripheral support structure (501);

7. The piercing device (550) of claim 6, further comprising a piercing element (502) connected to the piercing element carrier (508, said the piercing element (502) preferably being arranged to remain within the inner volume (503) at the initial position and to protrude from the inner volume (503) at the stop position.

8. A testing device (790), comprising
- a piercing device (750) according to claims 6 or 7;
- a fluid sample receiving unit (752) for receiving a fluid sample for testing;
- at least one fluid transport element (754, 756) having an input end fluidly connected to the fluid sample receiving unit, the fluid transport element arranged and configured to transport a fluid away from the input end; and
- at least one testing unit (758, 760) in fluidic communication with the fluid transport element, the testing unit comprising a respective reacting material configured to react in a predetermined manner to a pre-specified analyte or property of the fluid.

9. The testing device (990) of claim 8, further comprising at least one solution chamber (962) containing a respective buffer solution, and flow control means (964.1, 954.2) configured to control a transfer of the buffer solution to the fluid transport element and/or to the fluid sample receiving unit.

10. The testing device (990) of claim 9, wherein the flow control means (964.1, 964.2) is configured to control a transfer of the buffer solution from the solution chamber (962) to the at least one fluid transport element (954) or/and to the fluid sample receiving unit (952) either:
- before a fluid sample is transferred to the at least one fluid transport element or/and to the fluid sample receiving unit; or
- while the fluid sample is being transferred to the at least one fluid transport element or/and to the fluid sample receiving unit; or
- after the fluid sample has been transferred to the at least one fluid transport element or/and to the fluid sample receiving unit; or
- any combination thereof.

11. The testing device according to at least one of claims 8 to 10, further comprising:
- an optical sensor, arranged and configured to detect light reflected from the at least one testing unit and to convert the detected light into an electrical signal representing an intensity and/or a color of the detected light,
- a conversion unit for converting the electrical signal into digital data representing the intensity and/or the color of the detected light, and
- a transmitter unit for wirelessly transmitting the digital data.

12. Method for actuating a hand-operated actuator mechanism for driving an external piercing element, the method comprising:
- applying a predetermined pushing finger force on a pushing member, thereby breaking a first breakable connection element connecting a mounting element to a trigger element;
- moving a piercing element carrier connected via a second breakable connection element to the trigger element from an initial position wherein the first and the second breakable connection elements are unbroken, to a stop position wherein an abutment (116) stops a movement of the piercing element carrier.

13. The method of claim 12, further comprising:
- breaking the second breakable connection element when the piercing element carrier reaches the stop position;
- acting on the piercing element carrier for returning the piercing element carrier from the stop position in the direction of the initial position.

14. The method of claim 12 or 13, wherein breaking the first breakable connection element requires applying a pushing finger force of 20-100 N.

15. A method for operating a testing device, the method comprising:
- performing the steps of any one of claims 12 to 14;
- transporting a fluid via a fluid transport element from a piercing element to at least one testing unit;
- reacting a respective reacting material in a predetermined manner to a pre-specified analyte or property of the fluid.

## Patentansprüche

1. Handbetätigter Betätigungsmechanismus (100) zum Antreiben eines externen Stechelements (102) mit einer vordefinierten Kraft, wobei der Betätigungsmechanismus (100) Folgendes umfasst
- ein Befestigungselement (104);
- ein Auslöseelement (106);
- einen Stechelementträger (108) für ein externes Stechelement, wobei
- das Auslöseelement (106) mit dem Befestigungselement (104) über ein erstes brechbares Verbindungselement (110) verbunden ist, das so ausgelegt ist, dass es bricht, wenn eine vordefinierte Kraft auf das Auslöseelement ausgeübt wird, wobei das Auslöseelement (106) ferner ein Drückelement (112) umfasst oder mit diesem verbunden ist, das mit einem menschlichen Finger gedrückt werden kann und das so angeordnet ist, dass es eine Fingerkraft über das Auslöseelement auf das erste zerbrechliche Verbindungselement überträgt, um ein Brechen des ersten zerbrechlichen Verbindungselements zu bewirken, wenn eine das Drückelement (112) drückende Fingerkraft (F) einen vorbestimmten Schwellenwert überschreitet; und wobei
- der Stechelementträger (108) mit dem Auslöseelement (106) über ein zweites brechbares Verbindungselement (114) verbunden ist, wobei der Stechelementträger zwischen einer Ausgangsposition, in der das erste und das zweite brechbare Verbindungselement (110, 114) ungebrochen sind, und einer Stopp-Position, in der ein Anschlag (116) eine Bewegung des Stechelementträgers (108) stoppt, bewegbar ist.

2. Betätigungsmechanismus (200) nach Anspruch 1, wobei das zweite brechbare Verbindungselement (214) so konfiguriert ist, dass es bricht, wenn der Stechelementträger (208) die Anschlagposition erreicht.

3. Betätigungsmechanismus (300) nach Anspruch 1 oder 2, ferner umfassend ein elastisches Element (318), das so angeordnet ist, dass es zum Zurückbringen des Stechelementträgers aus der Anschlagposition in Richtung der Ausgangsposition auf den Stechelementträger einwirkt.

4. Betätigungsmechanismus (400) nach Anspruch 3, wobei das elastische Element (418) in einem gespannten oder komprimierten Zustand der Anschlag (416) ist.

5. Betätigungsmechanismus nach mindestens einem der vorhergehenden Ansprüche, wobei die erste brechbare Verbindung so beschaffen ist, dass sie bei Ausüben einer Druckfingerkraft von 20-100 N auf das Druckelement gebrochen wird.

6. Einstechvorrichtung (550), die Folgendes umfasst:
- eine periphere Tragstruktur (501), die ein Innenvolumen (503) bildet;
- einen handbetätigten Betätigungsmechanismus (500) nach einem der vorhergehenden Ansprüche, wobei das Befestigungselement mit der peripheren Tragstruktur (501) verbunden ist;

7. Einstechvorrichtung (550) nach Anspruch 6, die ferner ein Einstechelement (502) umfasst, das mit dem Einstechelementträger (508) verbunden ist, wobei das Einstechelement (502) vorzugsweise so angeordnet ist, dass es in der Ausgangsposition innerhalb des Innenvolumens (503) verbleibt und in der Stopp-Position aus dem Innenvolumen (503) herausragt.

8. Testvorrichtung (790), umfassend
- eine Einstechvorrichtung (750) nach einem der Ansprüche 6 oder 7;
- eine Flüssigkeitsproben-Aufnahmeeinheit (752) zur Aufnahme einer Flüssigkeitsprobe zur Prüfung;
- mindestens ein Fluidtransportelement (754, 756) mit einem Eingangsende, das in Fluidverbindung mit der Fluidprobenaufnahmeeinheit steht, wobei das Fluidtransportelement so angeordnet und konfiguriert ist, dass es ein Fluid von dem Eingangsende weg transportiert; und
- mindestens eine Testeinheit (758, 760), die in Fluidverbindung mit dem Fluidtransportelement steht, wobei die Testeinheit ein entsprechendes Reaktionsmaterial umfasst, das so konfiguriert ist, dass es in einer vorbestimmten Weise auf einen vorgegebenen Analyten oder eine vorgegebene Eigenschaft des Fluids reagiert.

9. Testvorrichtung (990) nach Anspruch 8, ferner umfassend mindestens eine Lösungskammer (962), die eine entsprechende Pufferlösung enthält, und eine Flusssteuerungseinrichtung (964.1, 954.2), die so konfiguriert ist, dass sie einen Transfer der Pufferlösung zu dem Fluidtransportelement und/oder zu der Fluidprobenaufnahmeeinheit steuert.

10. Testvorrichtung (990) nach Anspruch 9, wobei die Flusssteuerungseinrichtung (964.1, 964.2) so konfiguriert ist, dass sie einen Transfer der Pufferlösung von der Lösungskammer (962) zu dem mindestens einen Fluidtransportelement (954) oder/und zu der Fluidprobenaufnahmeeinheit (952) steuert:
- bevor eine Flüssigkeitsprobe zu dem mindestens einen Flüssigkeitstransportelement oder/und zu der Flüssigkeitsprobenaufnahmeeinheit übertragen wird; oder
- während die Flüssigkeitsprobe zu dem mindestens einen Flüssigkeitstransportelement oder/und zu der Flüssigkeitsprobenaufnahmeeinheit transportiert wird; oder
- nachdem die Flüssigkeitsprobe zu dem mindestens einen Flüssigkeitstransportelement oder/und zu der Flüssigkeitsprobenaufnahmeeinheit transportiert wurde; oder
- eine beliebige Kombination davon.

11. Testvorrichtung nach mindestens einem der Ansprüche 8 bis 10, ferner umfassend:
- einen optischen Sensor, der so angeordnet und konfiguriert ist, dass er von der mindestens einen Testeinheit reflektiertes Licht erfasst und das erfasste Licht in ein elektrisches Signal umwandelt, das eine Intensität und/oder eine Farbe des erfassten Lichts repräsentiert,
- eine Umwandlungseinheit zur Umwandlung des elektrischen Signals in digitale Daten, die die Intensität und/oder die Farbe des erfassten Lichts repräsentieren, und
- eine Sendeeinheit zur drahtlosen Übertragung der digitalen Daten.

12. Verfahren zur Betätigung eines handbetätigten Antriebsmechanismus zum Antrieb eines externen Stechelements, wobei das Verfahren umfasst:
- Aufbringen einer vorbestimmten Drückfingerkraft auf ein Drückelement, wodurch ein erstes brechbares Verbindungselement, das ein Befestigungselement mit einem Auslöseelement verbindet, gebrochen wird;
- Bewegen eines Stechelementträgers, der über ein zweites brechbares Verbindungselement mit dem Auslöseelement verbunden ist, aus einer Ausgangsposition, in der das erste und das zweite brechbare Verbindungselement ungebrochen sind, in eine Stopp-Position, in der ein Anschlag (116) eine Bewegung des Stechelementträgers stoppt.

13. Verfahren nach Anspruch 12, ferner umfassend:
- Brechen des zweiten brechbaren Verbindungselements, wenn der Stechelementträger die Anschlagposition erreicht;
- Einwirken auf den Stechelementträger, um den Stechelementträger aus der Anschlagposition in Richtung der Ausgangsposition zurückzuführen.

14. Verfahren nach Anspruch 12 oder 13, wobei das Brechen des ersten brechbaren Verbindungselements das Aufbringen einer Fingerdruckkraft von 20-100 N erfordert.

15. Verfahren zum Betreiben einer Prüfvorrichtung, wobei das Verfahren umfasst:
- das Durchführen der Schritte nach einem der Ansprüche 12 bis 14;
- Transport eines Fluids über ein Fluidtransportelement von einem Einstechelement zu mindestens einer Testeinheit;
- Reaktion eines entsprechenden Reaktionsmaterials in vorbestimmter Weise mit einem vorgegebenen Analyt oder einer vorgegebenen Eigenschaft der Flüssigkeit.

## Revendications

1. Un mécanisme d'actionnement manuel (100) pour entraîner un élément de perforation externe (102) selon une force prédéfinie, ledit mécanisme d'actionnement (100) comprenant :
- un élément de montage (104) ;
- un élément de déclenchement (106) ;
- un support d'élément de perforation (108) pour un élément de perforation externe, où
- l'élément de déclenchement (106) est relié à l'élément de montage (104) par un premier élément de jonction cassable (110) conçu pour se rompre lorsqu'une force prédéfinie est appliquée à l'élément de déclenchement, ledit élément de déclenchement (106) comprenant en outre ou étant relié à un élément de poussée (112) qui peut être poussé par un doigt humain et qui est conçu pour transférer la force du doigt au premier élément de jonction cassable via l'élément de déclenchement de manière à provoquer la rupture du premier élément de jonction cassable lorsqu'une pression exercée par la force du doigt (F) sur ledit élément de poussée (112) dépasse un seuil prédéfini ; et dans lequel
- le support d'élément de perforation (108) est relié à l'élément de déclenchement (106) par un second élément de jonction cassable (114), ledit support d'élément de perforation pouvant être déplacé entre sa position initiale, dans laquelle le premier et le second élément de jonction cassables (110, 114) demeurent intacts, et une position d'arrêt, dans laquelle une butée (116) vient stopper le mouvement du support d'élément de perforation (108).

2. Le mécanisme d'actionnement (200) de la revendication 1, au sein duquel le second élément de jonction cassable (214) est configuré pour être cassé lorsque le support d'élément de perforation (208) atteint sa position d'arrêt.

3. Le mécanisme d'actionnement (300) de la revendication 1 ou 2 comprenant également un élément élastique (318) conçu pour agir au niveau support d'élément de perforation (308) afin de faire revenir le support d'élément de perforation depuis sa position d'arrêt vers sa position initiale.

4. Le mécanisme d'actionnement (400) de la revendication 3, où la butée (416) constitue l'élément élastique (418) dans un état de tension ou de compression.

5. Le mécanisme d'actionnement selon au moins une des revendications précédentes, dans lequel le premier élément de jonction cassable est conçu pour être rompu lors de l'exercice d'une force de poussée de 20 à 100 N sur l'élément de poussée.

6. Un dispositif de perforation (550) comprenant :
- une structure de support périphérique (501) qui forme un volume intérieur (503) ;
- un mécanisme d'actionnement manuel (500) tel que défini par l'une quelconque des revendications précédentes ; où l'élément de montage est relié à la structure de support périphérique (501) ;

7. Le dispositif de perforation (550) prévu par la revendication 6, et comprenant en outre un élément de perforation (502) relié au support d'élément de perforation (508), ledit élément de perforation (502) étant de préférence agencé de façon à rester au sein du volume intérieur (503) en position initiale et à faire saillie depuis le volume intérieur (503) en position d'arrêt.

8. Un dispositif de test (790), comprenant
- un dispositif de perforation (750) conforme aux revendications 6 ou 7 ;
- une unité de réception d'échantillon de fluide (752) pour recevoir un échantillon de fluide à tester ;
- au moins un élément transporteur de fluide (754, 756) ayant une extrémité d'entrée reliée fluidiquement à l'unité de réception d'échantillons de fluide, l'élément transporteur de fluide étant disposé et configuré pour transporter un fluide depuis l'extrémité d'entrée ; et
- au moins une unité de test (758, 760) reliée fluidiquement à l'élément transporteur de fluide, cette unité de test renfermant un agent réactif respectif conçu pour réagir d'une manière prédéterminée à un analyte ou à une propriété du fluide spécifiés au préalable.

9. Le dispositif de test (990) de la revendication 8, comprenant en outre au moins un réservoir de solution (962) renfermant une solution tampon respective, et des dispositifs de contrôle du débit (964.1, 954.2) configurés pour réguler le transfert de la solution tampon vers l'élément transporteur de fluide et/ou vers l'unité de réception de l'échantillon de fluide.

10. Le dispositif de test (990) de la revendication 9, où les dispositifs de contrôle du débit (964.1, 964.2) sont configurés de façon à réguler le transfert de la solution tampon depuis le réservoir de solution (962) vers au moins un élément transporteur de fluide (954) ou/et vers l'unité de réception de l'échantillon de fluide (952) soit :
- avant le transfert d'un échantillon de fluide vers au moins un élément transporteur de fluide ou/et vers l'unité de réception de l'échantillon de fluide ; ou
- pendant le transfert d'un échantillon de fluide vers au moins un élément transporteur de fluide ou/et vers l'unité de réception de l'échantillon de fluide ; ou
- après le transfert d'un échantillon de fluide vers au moins un élément transporteur de fluide ou/et vers l'unité de réception de l'échantillon de fluide ; ou
- toute autre combinaison de ces paramètres.

11. Le dispositif de test conformément à au moins l'une des revendications 8 à 10, et comprenant en outre :
- un capteur optique, agencé et configuré de manière à détecter la lumière réfléchie par au moins une unité de test et convertir la lumière détectée en un signal électrique indiquant l'intensité et/ou la couleur de la lumière détectée,
- une unité de conversion permettant de convertir le signal électrique en données numériques représentant l'intensité et/ou la couleur de la lumière détectée, et
- une unité de transmission permettant un transfert sans fil des données numériques.

12. Méthode de mise en oeuvre d'un mécanisme d'actionnement manuel permettant le fonctionnement d'un élément de perforation externe, comme suit :
- appliquer une force prédéterminée de pression du doigt sur un élément de poussée, rompant ainsi un premier élément de jonction cassable qui relie un élément de montage à un élément de déclenchement ;
- déplacer un support d'élément de perforation relié par un deuxième élément de jonction cassable à l'élément déclencheur à partir d'une position initiale dans laquelle le premier et le deuxième élément de jonction cassables demeurent intacts, vers une position d'arrêt dans laquelle une butée (116) vient stopper le mouvement du support d'élément de perforation.

13. La méthode énoncée dans la revendication 12, prévoyant par ailleurs de :
- rompre le deuxième élément de jonction cassable lorsque le support d'élément de perforation atteint la position de butée ;
- agir sur le support d'élément de perforation pour ramener le support d'élément de perforation depuis sa position d'arrêt vers sa position initiale.

14. La méthode énoncée dans la revendication 12 ou 13, et dans laquelle la rupture du premier élément de jonction cassable nécessite l'application d'une force de poussée du doigt comprise entre 20 et 100 N.

15. Méthode pour l'utilisation d'un dispositif de test, définie comme suit :
- effectuer les étapes prévues par l'une quelconque des revendications 12 à 14 ;
- transporter un fluide via un élément transporteur de fluide depuis un élément de perforation et jusqu'à au moins une unité de test ;
- faire réagir un agent réactif respectif selon une méthode prédéterminée à un analyte ou à une propriété du fluide définis au préalable.
